# EUROPÄISCHE PATENTSCHRIFT

(11) **EP 0 697 404 B1**
(45) Veröffentlichungstag und Bekanntmachung des Hinweises auf die Patenterteilung: **18.04.2001**
(21) Anmeldenummer: 95112640.8
(22) Anmeldetag: 11.08.1995
(51) Int. Cl.: C07D 213/36, C07D 213/127, C07B 57/00

(54) **Neue enantiomerenreine Pyridylcycloalkylethylamine und deren Salze sowie Verfahren zu ihrer Herstellung**
New enantiomerically pure pyridyl cycloalkylethyl amines and their salts as well as process for their production
Pyridyl-cycloalkyl-ethylamines énantiomeriquement purs et leur sels ainsi qu'un procédé pour les préparer

(30) Priorität: 12.08.1994 DE 4428531
(43) Veröffentlichungstag der Anmeldung: 21.02.1996
(73) Patentinhaber: Boehringer Ingelheim Pharma KG, 55218 Ingelheim am Rhein (DE)
(72) Erfinder: Langbein, Adolf, Dr., D-55435 Gau-Algesheim (DE); Schneider, Heinrich, Dr., D-55218 Ingelheim am Rhein (DE); Bressler, Gerd-Rainer, D-55411 Bingen am Rhein (DE)
(74) Vertreter: Laudien, Dieter, Dr.

(56) Entgegenhaltungen:
- EP-A- 0 535 521
- ORG.PREP.PROCEED.INT., Bd. 24, Nr. 1, 1992 Seiten 87-91, MIAO,C.K. ET AL. 'The resolution of Racemic Amines by the Formation of Diastereomeric amides with Amico Acids'
- CHEMICAL ABSTRACTS, vol. 119, no. 7, 1993 Columbus, Ohio, US; abstract no. 72857h, MI,A. ET AL. 'Stereoselective Synthesis via Chiral Pinanone Amine ' Seite 1091; & YOUJI HUAXUE, Bd. 13, Nr. 2, 1993 Seiten 199-201, MI,A. ET AL. 'Stereoselective Synthesis via Chiral Pinanone Imine '
- TETRAHEDRON LETT., Bd. 34, Nr. 14, 1993 OXFORD, Seiten 2259-2263, MIAO,C.K. ET AL. 'A simple and Effective Enantiomeric Synthesis of a Chiral Primary Amine '
- J.MED.CHEM., Bd. 37, Nr. 7, 1.April 1994 WASHINGTON, Seiten 913-923, LAZER,E.S. ET AL. 'Benzoxazolamines and Benzoxathiazolamines : Potent, Enantioselective Inhibitors of Leucotriene Biosynthesis with a Novel Mechanism of Action '
- SYNTHESIS, Oktober 1992 STUTTGART, Seiten 972-976, FALORNI,M. ET AL. 'Chiral Ligands Containing Heteroatoms; 9. General Procedure for the Synthesis of Optically Active 1-(2-Pyridyl)alkylamines from alpha-Amino Acids '

## Beschreibung

Die vorliegende Erfindung betrifft ein Verfahren zur Herstellung enantiomerenreiner Pyridylcycloalkylethylamine der allgemeinen Formel 1a und 1b, - worin n eine ganze Zahl 2, 3, 4, 5, 6 oder 7 verkörpert -, deren Salze sowie Verfahren zu deren Herstellung. 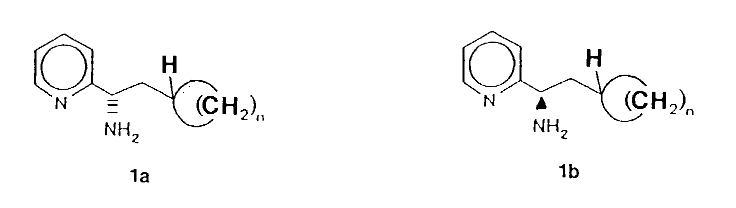 Derartige Amine verkörpern wichtige Edukte für die Synthese von kondensierten Oxazol- und Thiazolderivaten, die Leukotrien-Biosyntheseinhibitoren darstellen, und aufgrund dieser Eigenschaft als wirksame Arzneimittel bei der Bekämpfung verschiedener Krankheiten - u.a. von Asthma - eingesetzt werden können. Verbindungen dieses Typs sind bereits aus dem Stand der Technik bekannt [Europäische Patentanmeldung 0 535 521].

Aus dem Stand der Technik sind ebenfalls Verfahren zur Herstellung derartiger Amine bekannt. So besteht eine Möglichkeit darin, durch Umsetzung von 2-Pyridin-carbaldehyd mit einem geeigneten Aminoalkohol das in 4-Stellung entsprechend substituierte 2-Pyridyl-1,3-oxazolidinderivat zugänglich zu machen, das mit seinem Imin im Gleichgewicht steht (vgl. nachfolgendes Formelschema). Durch die weitere Umsetzung mit einem Cyclohexylmethylmagnesiumhalogenid im Rahmen einer Grignard-Addition sind die entsprechenden diastereomeren Cycloalkyl-pyridylmethylamine herstellbar, welche nach der oxidativen Abspaltung des derivatisierten Hydroxyethylsubstituenten das gewünschte chirale Amin liefern. Das Verhältnis der beiden diastereomeren Amine beträgt dabei 87:13 - im Falle der Substituent Y *iso*-Propyl bedeutet. 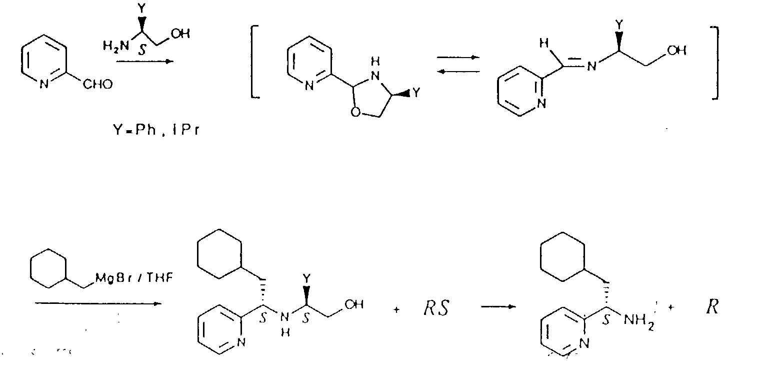 Eine alternative Syntheseroute geht von einem Cyclohexyl-methyl-2-pyridylketon aus, das in einer Kondensationsreaktion mit einem Valinol zu den entsprechend substituierten 1,3-Oxazolidin-Derivat umgesetzt wird, welches mit seinem korrespondierenden Imin wiederum im Gleichgewicht steht (vgl. nachfolgendes Formelschema). Die katalytische Hydrierung der Iminfunktion führt zu den entsprechenden diastereomeren Aminen. Ausgehend von einem R-Aminoalkohol ergibt sich dabei beispielsweise ein Verhältnis der beiden Diastereomeren SR:RR von 98:2 - im Falle der Substituent Y einen Phenylrest verkörpert. - Die nachfolgende oxidative Spaltung mit Natriumperjodat führt im abschließenden Reaktionsschritt zu den gewünschten Aminen [C.K. Miao, R. Sorcek und P.-J. Jones, Tetrahedron Lett. 34 (14), 2259]. 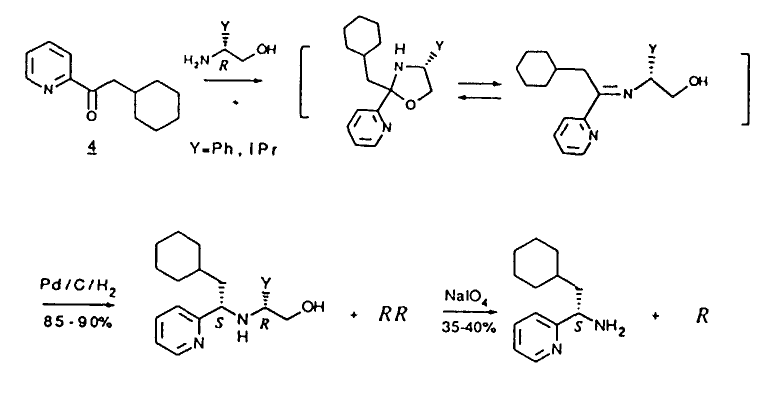 Daneben ist ein weiteres Verfahren aus dem Stand der Technik bekannt, bei dem das racemische Gemisch von α-(Cyclohexylmethyl)-2-pyridylmethylaminen über die Stufe der entsprechenden diastereomeren Amide (durch Umsetzung mit geeigneten Aminosäuren) getrennt werden [C. K. Miao, R. Sorcek und J. H. Nagel, Org. Prep. Proced. Int. 24 (1), 1987].

Der Nachteil aller aus dem Stand der Technik bekannten Verfahren besteht insbesondere darin, daß die bei den jeweiligen Reaktionsfolgen anfallenden enantiomeren Amine durch zeit- und kostenaufwendige Verfahren - z.B. durch mehrmalige Kristallisation des Racemats - in die einzelnen Enantiomere getrennt werden.

Die Aufgabe der vorliegenden Erfindung besteht somit darin, die Nachteile der aus dem Stand der Technik bekannten Verfahren zu überwinden.

Erfindungsgemäß wird diese Aufgabe dadurch gelöst, daß man im ersten Reaktionsschritt Cyanpyridin (2) mit einem Cycloalkylhalogenmethan der allgemeinen Formel 3 im Rahmen einer Grignardreaktion umsetzt. 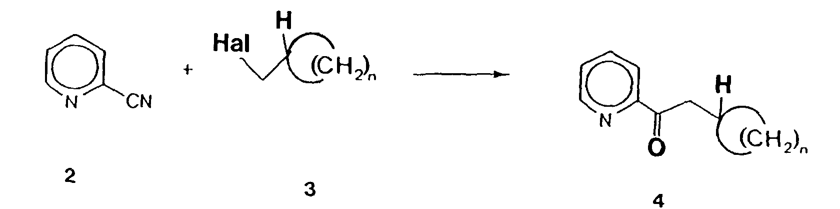 Derartige Reaktionen sind an sich aus dem Stand der Technik bekannt [J. March, Advanced Organic Chemistry, 3^{rd} Edition, John Wiley & Sons, New York 1985, S. 828 und zit. Lit.]. Als Halogenverbindungen werden dabei bevorzugt die entsprechenden Bromcycloalkane eingesetzt. Die Additionsreaktion des Grignard-Reagenzes wird zweckmäßigerweise in inerten Reaktionsmedien durchgeführt, worunter Kohlenwasserstoffe, wie z.B. Petroletherfraktionen oder Alkylaromaten- wie z.B. Toluol - oder Dialkylether - wie z.B. Diethylether - bevorzugt werden. Ganz besonders bevorzugt wird Methyl-tert.-butylether als Reaktionsmedium eingesetzt. Ebenso ist es möglich, Gemische der genannten Lösungsmittel einzusetzen.

Die Reaktionstemperatur ist in weiten Grenzen wählbar und wird bei zu tiefen Temperaturen lediglich durch eine zu geringe Reaktionsgeschwindigkeit und bei zu hohen Temperaturen durch die Bildung von Neben- bzw. Zersetzungsprodukten sowie durch die physikalischen Eigenschaften des Reaktionsmediums eingeschränkt. Bevorzugt wird eine Reaktionstemperatur im Bereich von 40 bis 70°C, wobei das Temperaturintervall von 50 - 60°C besonders bevorzugt wird. Bevorzugt wird die Grignard-Reaktion in Gegenwart von Trialkylhalogensilanen durchgeführt, wobei Trimethylchlorsilan besonders bevorzugt wird.

Die auf diese Weise zunächst hergestellten Ketimine werden unter Zuhilfenahme von Säuren - vorzugsweise von verdünnten Mineralsäuren und besonders bevorzugt unter Verwendung von halbkonzentrierter Salz- oder Schwefelsäure - hydrolysiert.

Das aus der Hydrolysereaktion resultierende Keton der allgemeinen Formel 4 wird im nachfolgenden Reaktionsschritt mit enantiomerenreinen Phenylethylaminen vom Typ 5 unter den Reaktionsbedingungen einer azeotropen Dehydratisierung d. h. unter Rückflußbedingungen mit zu einer Azeotropbildung mit Wasser befähigten Lösungsmitteln, wie z.B. halogenierte Kohlenwasserstoffe - beispielsweise Chloroform - oder Kohlenwasserstoffe - wie zum Beispiel Benzol oder Toluol, worunter Toluol besonders bevorzugt wird, zu den entsprechenden Ketiminen der allgemeinen Formel 6 umgesetzt. Als Schleppmittel werden dabei halogenierte Kohlenwasserstoffe - wie zum Beispiel Dichlormethan oder Chloroform - oder Aromaten oder auch Alkylaromaten bevorzugt; besonders bevorzugt wird Toluol eingesetzt. 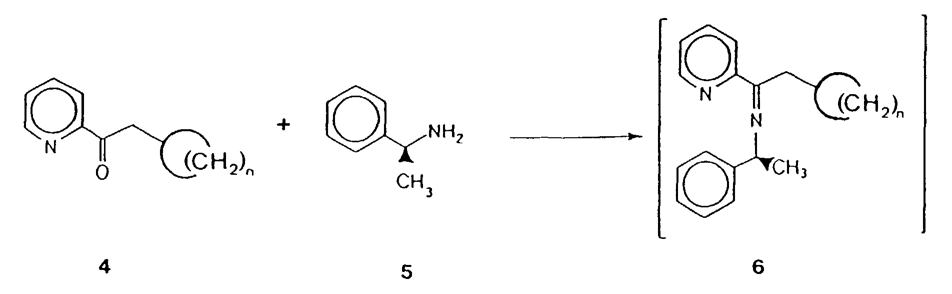 Die Hilfsstoffe, die eine derartige Dehydratisierung katalysieren, sind aus dem Stand der Technik wohlbekannt. Bevorzugt werden Sulfonsäurederivate eingesetzt, worunter das Hydrat der p-Toluolsulfonsäure besonders bevorzugt wird.

Besonders bevorzugt wird die Dehydratisierungsreaktion in Gegenwart von Kieselgel durchgeführt.

Nach erfolgter Dehydratisierung wird die Reaktionsmischung abgesaugt und im Vakuum - vorzugsweise im Wasserstrahlvakuum - eingeengt.

Zur Reduktion der so erhaltenen Ketimine der allgemeinen Formel 6 werden diese zunächst in einem unter reduktiven Reaktionsbedingungen inerten, polaren Lösungsmittel gelöst. Bevorzugt werden hierbei niedere Alkanole - wie z.B. C₁-C₆-Alkohole -, worunter Ethanol besonders bevorzugt wird. 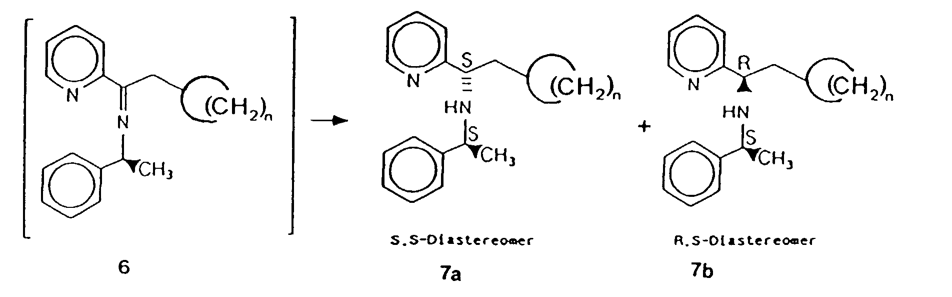 Die Reduktion von Ketiminen ist an sich aus dem Stand der Technik bekannt und erfolgt mit komplexen Hydriden. Dabei werden komplexe Hydride des Bors oder des Aluminiums bevorzugt. Besonders bevorzugt wird Natriumtetrahydridoboranat oder Lithiumtetrahydridoalanat als Reduktionsmittel eingesetzt [N.G. Gaylord, Reduktion with Complex Metal Hydrides, Wiley, New York 1965; A. Hàjos, Complex Hydrides, Elsevier, New York 1979; V. Bazant, M. 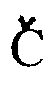 apka, M. 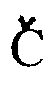 erny, V. Chvalovsky, K. Kochloefl, M. Kraus und J. Màlek, Tetrahedron Lett. 9, 3303].

Zweckmäßigerweise soll die Temperatur bei der Zugabe der Lösung des Reduktionsmittels - beispielsweise im Falle von Natriumborhydrid 10°C - nicht übersteigen. Nach erfolgter Zugabe wird die Reaktionslösung auf Raumtemperatur - d.h.: auf ca. 25°C - erwärmt und anschließend mit einer basisch reagierenden Verbindung - bevorzugt mit der wässerigen Lösung eines Alkalihydroxids, und besonders bevorzugt mit wässeriger Natronlauge (1N) - ein pH-Wert von größer als 8 - bevorzugt 9 - eingestellt. Nach dem Abdestillieren des Lösungsmittels wird der Rückstand mit einem unpolaren Lösungsmittel - bevorzugt mit einem Dialkylether, besonders bevorzugt Methyl-tert.-butylether - gegen Wasser ausgeschüttelt.

Das so erhaltene Gemisch aus den diastereomeren Aminen der allgemeinen Formeln 7a und 7b wird mit geeigneten Säuren - wie vorzugsweise Fumar- oder Oxalsäure, worunter Fumarsäure besonders bevorzugt wird - umgesetzt, wodurch eine Anreicherung des gewünschten Amins erzielt wird.

Zur Herstellung der diastereomeren Salze werden die Amine 7a und 7b zunächst in einem inerten Lösungsmittel gelöst. Als Lösungsmittel werden Niederalkylester (C₁ - C₆) niederer Carbonsäuren (C₁ - C₆) bevorzugt, worunter Essigsäureethylester besonders bevorzugt wird. Zu der leicht erwärmten Lösung wird bei einer Temperatur im Bereich von vorzugsweise 40 bis 60°C und besonders bevorzugt bei 50°C die Lösung der zur Salzbildung mit dem Amin 7a bzw. 7b geeigneten zweibasischen Säure (HXXH) in einem polaren Lösungsmittel zugegeben, wobei als Lösungsmittel niedere Alkanole - z.B. C₁ -C₆-Alkohole oder deren Mischungen - bevorzugt werden und Ethanol besonders bevorzugt wird. 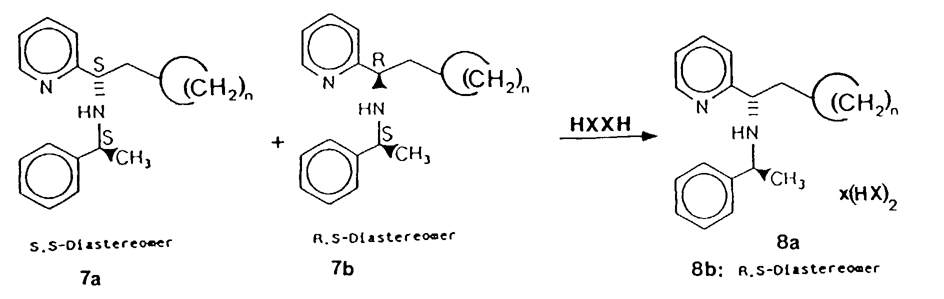 Das so hergestellte Salz 8 des gewünschten Diastereomeren wird isoliert und in einem polaren Lösungsmittel - bevorzugt einem niederen Alkanol, z.B. in einem C₁-C₆-Alkohol, besonders bevorzugt in Ethanol - bei einer Temperatur in einem Bereich von 50 bis 100°C - bevorzugt in einem Bereich von 50 bis 65°C, besonders bevorzugt in einem Intervall 55 bis 60°C - gelöst und in Gegenwart eines zur Wasserstofftransferkatalyse befähigten Katalysators mit einem Wasserstoffdonor umgesetzt. 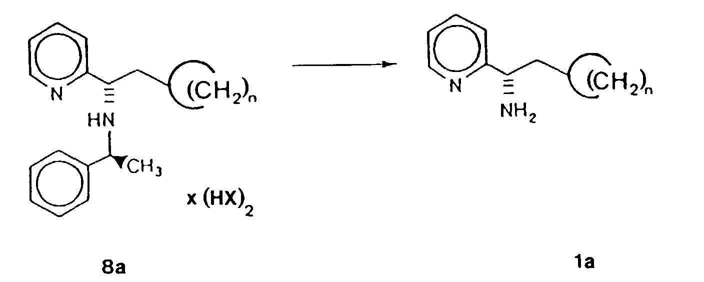 Derartige Katalysatoren und geeignete Wasserstoffdonoren sind ebenfalls an sich aus dem Stand der Technik bekannt [vgl. z.B. G. Brieger und T.J. Nestrick, Chem.Rev., 74 (1974) 567]. Als Katalysator wird Palladium auf Kohle bevorzugt, wobei ein derartiger Katalysator mit einem 10 %-igen Palladiumanteil besonders bevorzugt wird.

Als Wasserstoffdonor wird bevorzugt Cyclohexen eingesetzt.

Nach abgeschlossener Debenzylierung wird das Reaktionsgemisch auf eine Temperatur von ca. 50°C abgekühlt und der Katalysator abgetrennt und mit Wasser nachgewaschen. Die so erhaltene Lösung des enantiomeren Amins wird nach dem Einengen im Vakuum mit einem mit Wasser nicht mischbaren Extraktionsmittel extrahiert. Bevorzugt werden als Extraktionsmittel Dialkylether eingesetzt, worunter Methyl-tert.-butylether besonders bevorzugt wird.

Nach dem Waschen mit Wasser ist das Amin vom Typ 1 (im vorliegenden Beispiel 1a) beispielsweise in Form seines Succinates in die wässerige Phase übergegangen. Diese wird mit einer basisch reagierenden Verbindung - bevorzugt mit Ammoniaklösung und besonders bevorzugt mit konzentrierter Ammoniaklösung - alkalisch gestellt und mit einem mit Wasser nicht mischbaren Lösungsmittel - bevorzugt einem halogenierten Kohlenwasserstoff, besonders bevorzugt Dichlormethan - extrahiert. Nach dem Trocknen und Einengen der vereinigten Extrakte im Vakuum erhält man das gewünschte Amin als Rohprodukt, welches nach Lösen in einem polaren Lösungsmittel - bevorzugt in einem Keton und besonders bevorzugt in Aceton - beispielsweise mit Oxalsäure in das entsprechende - kristalline - Oxalat übergeführt werden kann.

Die eingangs genannten Aufgaben werden durch die in den folgenden Beispielen beschriebenen Verfahrensschritte gelöst. Verschiedenartige andere Ausgestaltungen des Verfahrens werden für den Fachmann aus der vorliegenden Beschreibung ersichtlich. Es wird jedoch ausdrücklich darauf hingewiesen, daß die Beispiele und die diesen zugeordnete Beschreibung lediglich zum Zweck der Erläuterung und Beschreibung vorgesehen und nicht als Einschränkung der Erfindung anzusehen sind.

Auf die angegebene Art und Weise läßt sich beispielsweise das 1-S-(2-Pyridyl)-2-cyclohexylethylamin in einem Anteil 96,1 % in Form seines Semi-Oxalates isolieren; jedoch kann das erfindungsgemäße Verfahren ebenso erfolgreich auch zur Synthese anderer gewünschter Enantiomere oder- Diastereome eingesetzt werden.

### Beispiel 1:

### (2-Pyridyl)-cyclohexylmethylketon

Zu einer Grignard-Lösung, die durch 18 bis 20-stündiges Rückflußkochen von 73 g (3 g-Atom) Magnesium und 532 g (3,0 Mol) Brommethylcyclohexan in 1540 ml Methyl-tert.-butylether unter Zusatz von 44 g (0,2 Mol) Trimethylsilylchlorid durch schwaches Rückflußkochen bei 52 - 58°C bereitet worden ist, fügt man 208 g (2 Mol) 2-Cyanpyridin - gelöst in 1200 ml Methyl-tert.-butylether - bei einer Temperatur im Bereich von 0 - 20°C zu und läßt 1 Stunde nachreagieren. Eine - eventuell entstehende - schwer rührbare Suspension wird mit weiteren 800 ml Methyl-tert.-butylether verdünnt. Die Mischung wird - nach erfolgter Umsetzung- mit 1500 ml Wasser bei 5°C zersetzt und dann mit 450 ml halbkonzentrierter Salzsäure bei 15-25°C unter Kühlung während einer Zeitspanne von ca. 30 Minuten behandelt. Nach 1 Stunde werden die Phasen getrennt. Die organische Phase wird mit Wasser gewaschen, getrocknet und am Rotationsverdampfer bei 40°C und bei einem Druck von ca. 500 mbar eingeengt.

Man erhält das Pyridylcyclohexylmethylketon als Rohöl in 108-109 % Rohausbeute mit einem GC-Gehalt von 80 - 89 %, was einer Ausbeute von 87 - 96 % der Theorie entspricht.

### Dünnschichtchromatographie

Kieselgelfertigplatten Merck Si 60 F 254
Laufmittel: Cyclohexan/Essigester = 80/20 (V/V)
Reagenz: UV-Licht 254 nm
Dragendorff-Reagenz

### Beispiel 2:

### 1-(S)-1-(1'-(S)-Phenylethan-1-yl)amino-1-(pyridin-2-yl)-2-cyclohexylethan ("S,S-Diastereomer")

207 g Pyridyl-cyclohexylmethylketon (GC = 89,2 %; entsprechend 0,91 Mol werden mit 136 g (1,12 Mol) S-(-)-1-Phenethylamin, 500 mg p-Toluolsulfonsäure Hydrat und 136 g Kieselgel Merck (0,063-0,2 mm) in 2100 ml Toluol 4 bis 5 Stunden am Wasserabscheider unter Rückfluß gekocht.

Dann wird die Reaktionsmischung abgesaugt und am Rotationsverdampfer eingeengt. Den Rückstand versetzt man mit 2100 ml Ethanol und gibt 38,6 (1.02 Mol) NaBH₄ in fester Form im Laufe von 40 min in die Lösung, wobei die Temperatur auf ca. 10°C gehalten wird. Die Mischung läßt man 3,5 Stunden nachreagieren, wobei die Innentemperatur auf +25°C ansteigt. Man stellt mit 60 ml 1N Natronlauge auf pH = 9 ein und destilliert das Ethanol bei 40°C und 80 mbar ab. Der Rückstand wird mit Methyl-tert.-butylether (800 ml + 400 ml) und Wasser (1800 ml) extrahiert, die organische Phase mit Natriumsulfat getrocknet, abgesaugt und eingeengt. Man isoliert 285 g Rohöl mit einem Enantiomerengehalt von SS : RS = 90 : 10 %.

### Dünnschichtchromatographie

Kieselgelfertigplatten Merck Si 60 F 254
Laufmittel: Cyclohexan / Essigester / Methanol = 80/20/3 (V/V/V)

Das Diastereomerengemisch wird als Rohöl (ca. 285 g) in 3990 ml Essigester bei Raumtemperatur unter Rühren gelöst und mit einer 50°C warmen Lösung von 107 g Fumarsäure (0,924 Mol) in 1800 ml Ethanol versetzt.

Man rührt 2 Stunden bei Raumtemperatur nach und saugt über eine Nutsche ab.

Die so isolierten Kristalle werden bei 60°C im Vakuumtrockenschrank unter einem Druck von ca. 120 mbar bis zur Gewichtskonstanz über einen Zeitraum von ca. 6 Stunden getrocknet.

| | |
|---|---|
| Ausbeute | 201,6 g S,S-Diastereomeres Fumarsalz (1:1) = 52,4 % bezogen auf das Pydridylketon |
| Schmelzpunkt | 98-102°C |
| Chirales HPLC | S,S-Enantiomer 99,7 % |

### Beispiel 3:

### 1-S-(2-Pyridyl)-2-cyclohexylethylamin

40 g (0,094 Mol) S,S-diastereomeres Fumarsalz (1:1) werden in 800 ml Ethanol bei 55 bis 60°C Innentemperatur in Lösung gebracht. Man fügt 80 g (0,94 Mol) Cyclohexen schnell dazu und versetzt mit einer Suspension aus 12,8 g Palladium-Kohle (10 % E 10 ND der Degussa AG) und 120 ml Wasser. Die Mischung wird 4 - 5 Stunden auf Rückflußtempetatur erhitzt. Anschließend kühlt man das Reaktionsgemisch auf eine Temperatur von 50°C ab und saugt vom Katalysator ab, der mit 20 ml entmineralisiertem Wasser nachgespült wird. Die klare Lösung wird am Rotationsverdampfer bei 40°C und 120 mbar bis zum öligen Rückstand eingeengt. Man extrahiert mit Methyl-*tert*.-butylether (2 x 100 ml) und wäscht mit Wasser (1 x 80 ml) nach. Danach befindet sich das Amin als Succinat in der wässerigen Phase.

Diese wird mit 15 ml konzentriertem Ammoniak alkalisch gestellt und mit Dichlormethan (2 x 100 ml) extrahiert. Nach dem Trocknen der vereinigten organischen Extrakte mit Na₂SO₄ wird abgesaugt und am Rotationsverdampfer bei 40°C und 600 mbar bis zum Verbleib eines öligen Rückstands eingeengt. Man erhält 11,9 g (0,0582 Mol) des Rohproduktes in Form eines Öls. Dieses wird in 150 ml Aceton gelöst und mit einer Lösung von 2,62 g Oxalsäure (0,0291 Mol) in 35 ml Aceton versetzt. Das ausgefallene neutrale Oxalat wird nach 1 Stunde Rühren abgesaugt, mit wenig Aceton kalt nachgewaschen (20 ml) und im Vakuumtrockenschrank bei 60°C und bei einem Druck von 100 mbar über einen Zeitraum von 5 Stunden getrocknet.

| | |
|---|---|
| Ausbeute | 11,92 g l-S-(2-Pyridyl)-2-cyclohexylethylamin x 1/2 Oxalat entsprechend 57,1 % d. Th. bezogen auf das eingesetzte Fumarat |
| Schmelzpunkt | 161 - 164°C |
| Chirales HPLC | Gehalt an l-S-(2-Pyridyl)-2-cyclohexylethylamin 96,1 % |

### Dünnschichtchromatographie

Kieselgelfertigplatten Merck Si 60 F 254
Laufmittel: Dichlormethan/Methanol/konz. Ammoniak 90/10/0,5 (V/V/V)
R_{f}-Wert: ca. 0,5 - 0,6

## Patentansprüche

1. Verfahren zur Herstellung von enantiomerenreinen Pyridylcycloalkylethylaminen der allgemeinen Formel 1a oder 1b, 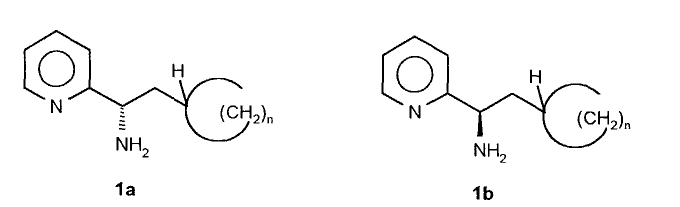 worin n eine ganze Zahl 2, 3, 4, 5, 6 oder 7 verkörpert, dadurch gekennzeichnet, daß man
a) Cyanpyridin (2) mit einem Halogenmethylcycloalkan der allgemeinen Formel 3 in Gegenwart von Magnesium in einem inerten Reaktionsmedium umsetzt und das Reaktionsgemisch nach erfolgter Umsetzung unter sauren Bedingungen hydrolisiert und das resultierende Keton der allgemeinen Formel 4 isoliert; 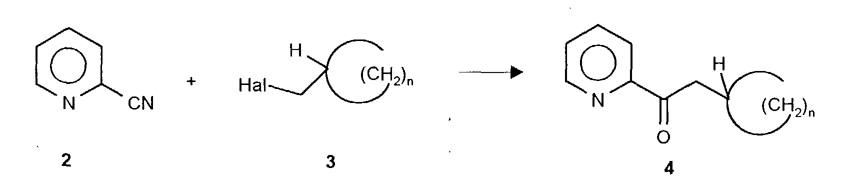
b) das aus der Grignardreaktion resultierende Keton der allgemeinen Formel 4 mit R- bzw. S-Phenylethylamin (5) im Rahmen einer Dehydratisierungsreaktion ggf. in Gegenwart eines die Dehydratisierung katalysierenden Stoffes umsetzt und das resultierende Ketimin der allgemeinen Formel 6 isoliert; 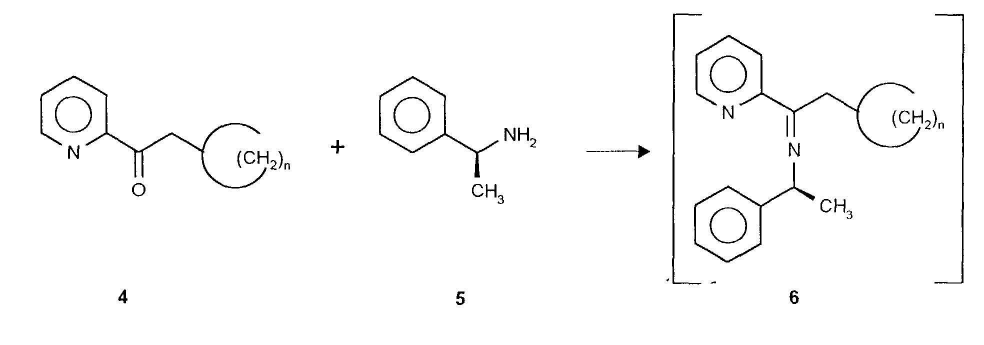
c) das aus der Kondensationsreaktion resultierende Ketimin der allgemeinen Formel 6 in einem inerten, polaren Lösungsmittel mit einem Reduktionsmittel umsetzt, nach erfolgter Reduktion mit der wässerigen Lösung einer alkalisch reagierenden Verbindung einen pH-Wert von größer als 8 einstellt und die aus der Reduktion resultierenden Diastereomere der allgemeinen Formel 7a und 7b aus dem Reaktionsgemisch mittels eines unpolaren Lösungsmittels extrahiert und in Form ihres Diastereomerengemisches isoliert; 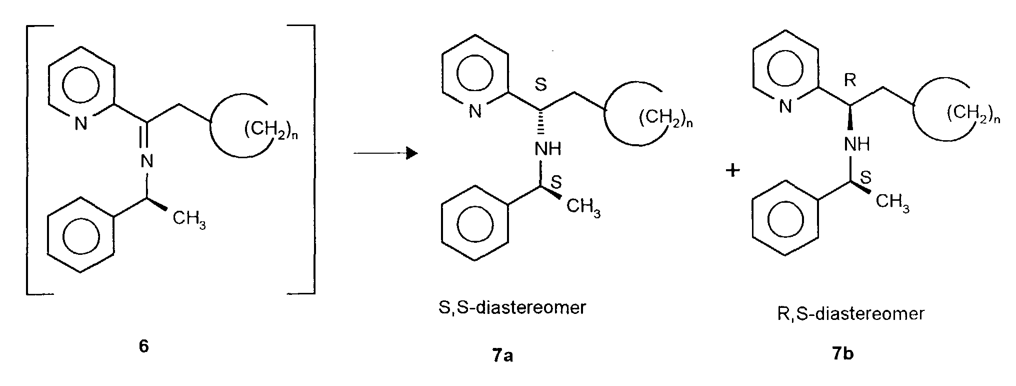
d) das so erhaltene Diastereomerengemisch der Amine der allgemeinen Formel 7a und 7b mit einer geeigneten Säure in einem inerten Lösungsmittel umsetzt, wobei die zur Salzbildung mit dem jeweiligen Amin befähigte Säure vor der Zugabe zu der Lösung des Diastereomerengemischs in einem niederen Alkohol (C₁ - C₆) löst und so die Amine 7a und 7b in die ensprechenden diastereomeren Salze überführt und somit eine Anreicherung des jeweiligen gewünschten diastereomeren Salzes erzielt und das entsprechende Diastereomere in Form seines Salzes der allgemeinen Formel 8a oder 8b isoliert; 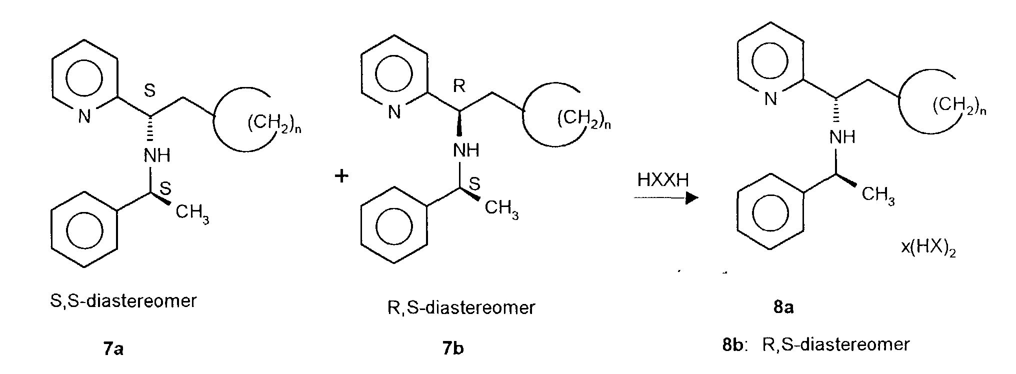
e) das auf diesem Wege hergestellte diastereomere Salz 8a oder 8b in einem polaren Lösungsmittel löst oder suspendiert und bei einer Temperatur im Bereich von 50 bis 100°C in Gegenwart eines zur Wasserstofftransferkatalyse befähigten Katalysators mit einem Wasserstoffdonor umsetzt und das so debenzylierte Amin der allgemeinen Formel 1a oder 1b mit einer basisch reagierenden Verbindung aus seinem Salz freisetzt und isoliert; 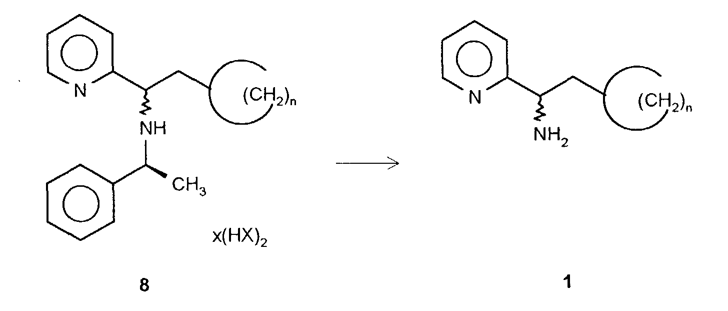
f) das so hergestellte enantiomere Amin der allgemeinen Formel 1a oder 1b gewünschtenfalls in einem polaren Lösungsmittel löst und sein Salz mit einer Mineral- oder einer organischen Säure in das entsprechende Salz überführt und isoliert.

2. Verfahren nach Anspruch 1, dadurch gekennzeichnet, daß man
a) Cyanpyridin (2) mit einem Halogenmethylcycloalkan der allgemeinen Formel 3 in Gegenwart von Magnesium in einem Kohlenwasserstoff oder in einem Dialkylether bei einer Temperatur in einem Intervall von 40 - 70°C gegebenenfalls in Gegenwart eines Trialkylhalogensilans umsetzt und das Reaktionsgemisch nach erfolgter Umsetzung mittels einer verdünnten Mineralsäure hydrolysiert und das resultierende Keton der allgemeinen Formel 4 isoliert; 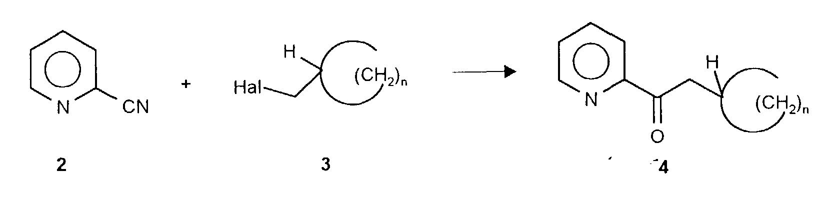
b) das aus der Grignardreaktion resultierende Keton der allgemeinen Formel 4 mit R- bzw. S-Phenylethylamin (5) im Rahmen einer Dehydratisierungsreaktion unter Rückflußbedingungen in einem zu einer Azeotropbildung mit Wasser befähigten Lösungsmittel - bevorzugt einem Halogenalkan oder einem aromatischen Kohlenwasserstoff - in Gegenwart eines die Kondensationsreaktion katalysierenden Sulfonsäurederivats oder in Gegenwart von Kieselgel umsetzt und das daraus resultierende Ketimin der allgemeinen Formel 6 isoliert; 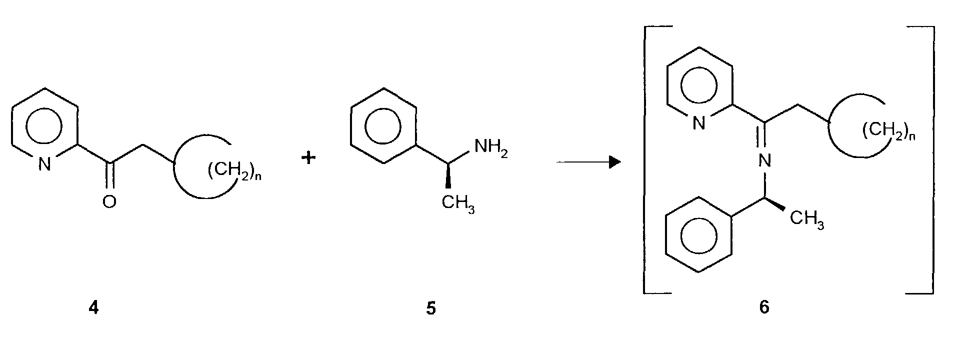
c) das aus der Kondensationsreaktion resultierende Ketimin der allgemeinen Formel 6 in einem Alkohol löst und mit einem komplexen Hydrid des Bors oder des Aluminiums bei einer Temperatur unterhalb von 10°C umsetzt, mit der wässerigen Lösung eines Alkalihydroxids einen pH-Wert von größer als 8 einstellt, und die aus der Reduktionsreaktion resultierenden Diastereomeren der allgemeinen Formel 7a und 7b aus der Reaktionsmischung mit einem Dialkylether extrahiert und in Form ihres Diasteromerengemisches isoliert; 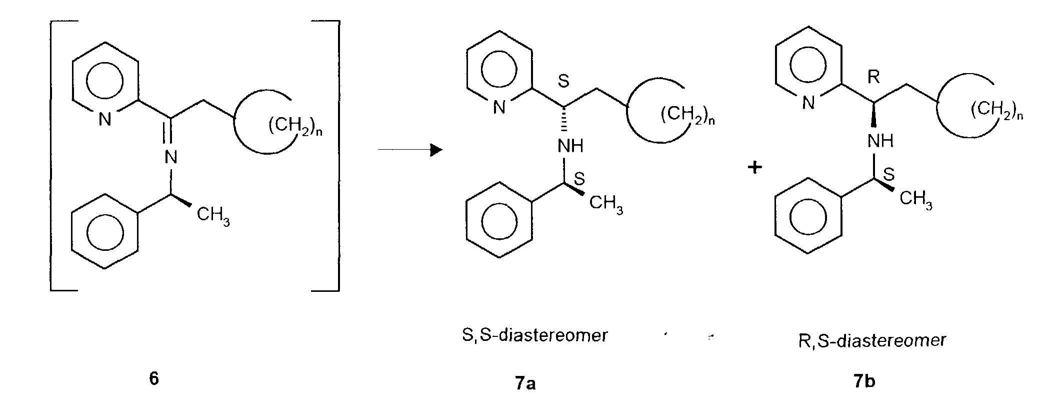
d) das so erhaltene Diastereomerengemisch der Amine mit der allgemeinen Formel 7a und 7b mit einer organischen Säure in einem Niederalkylester einer niederen Carbonsäure bei einer Temperatur in einem Intervall von 40 - 60°C umsetzt, wobei die zur Salzbildung mit dem jeweiligen Amin befähigte Säure vor der Zugabe zu der Lösung des Diastereomerengemischs in einem niederen Alkohol (C₁ - C₆) gelöst wird und die diastereomeren Amine der allgemeinen Formel 7a und 7b in die entsprechenden diastereomeren Salze überführt und somit eine Anreicherung des gewünschten diastereomeren Salzes erzielt und das entsprechende Diastereomer in Form seiner Salze der allgemeinen Formel 8a oder 8b isoliert; 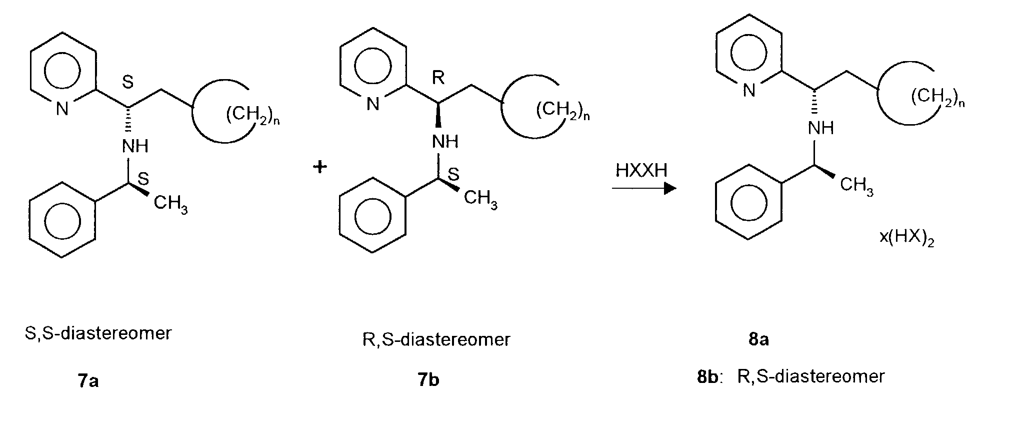
e) das auf diesem Wege hergestellte diastereomere Salz der allgemeinen Formel 8a oder 8b in einem niederen Alkanol (C₁ - C₆-Alkohol) löst oder suspendiert und bei einer Temperatur im Bereich von 50 bis 65°C löst und in Gegenwart eines zur Wasserstofftransferkatalyse befähigten Katalysators von Platin auf Kohle mit einem Wasserstoffdonor umsetzt und das debenzylierte Amin der allgemeinen Formel 1a oder 1b mit einer stickstoffhaltigen Base aus seinem Salz freisetzt und isoliert; 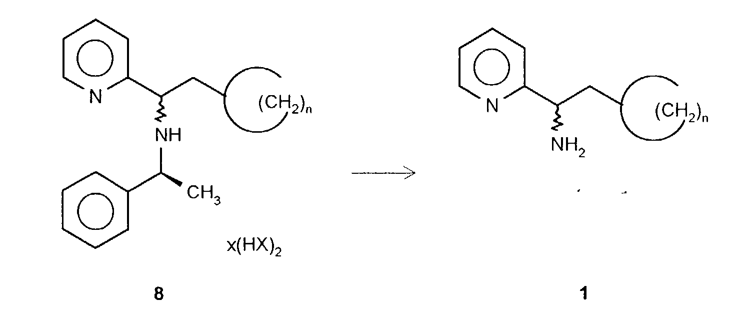
f) das so hergestellte enantiomere Amin der allgemeinen Formel 1a oder 1b gewünschtenfalls in einem Keton löst und mit einer Carbonsäure in das entsprechende Salz überführt und isoliert.

3. Verfahren nach Anspruch 2, dadurch gekennzeichnet, daß man
a) Cyanpyridin (2) mit einem Brommethylcycloalkan der allgemeinen Formel 3 in Methyl.-*tert*.-butylether als Reaktionsmedium bei einer Temperatur in einem Intervall von 50 bis 60°C in Gegenwart von Trimethylchlorsilan umsetzt und das Reaktionsgemisch nach erfolgter Umsetzung mittels halbkonzentrierter Chlorwasserstoff- oder Schwefelsäure hydrolysiert und das resultierende Keton der allgemeinen Formel 4 isoliert; 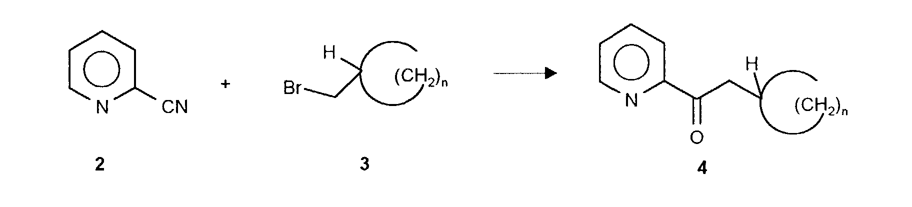
b) das aus der Grignard-Reaktion resultierende Keton der allgemeinen Formel 4 mit R- bzw. S-Phenylethylamin (5) im Rahmen einer Kondensationsreaktion unter Anwendung azeotroper Destillation mit Toluol und in Gegenwart von Kieselgel dehydratisiert und das aus dieser Kondensationsreaktion resultierende Ketimin der allgemeinen Formel 6 isoliert; 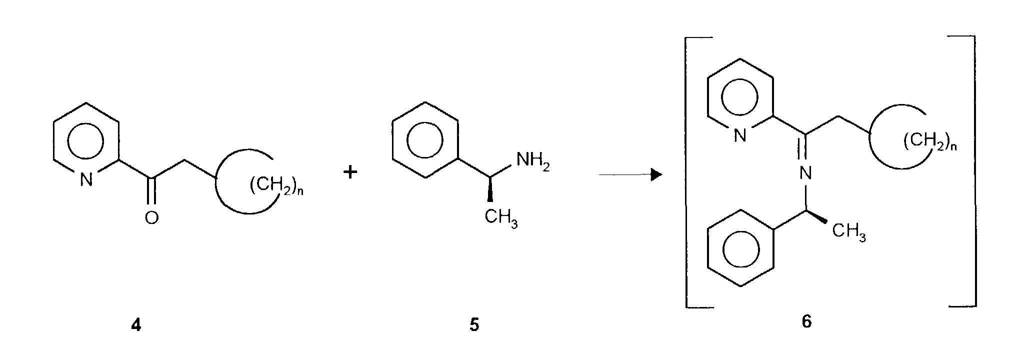
c) das aus der Kondensationsreaktion resultierende Ketimin der allgemeinen Formel 6 in Ethanol löst und mit Natriumtetrahydridoboranat oder Lithiumtetrahydroalanat bei einer Temperatur unterhalb von 10°C umsetzt, nach erfolgter Umsetzung mit wässeriger Natronlauge einen pH-Wert von größer als 9 einstellt und die aus der Reduktionsreaktion resultierenden diastereomeren Amine der allgemeinen Formel 7a und 7b mit Methyl*-tert.*-butylether extrahiert und in Form ihres Diastereomerengemischs isoliert; 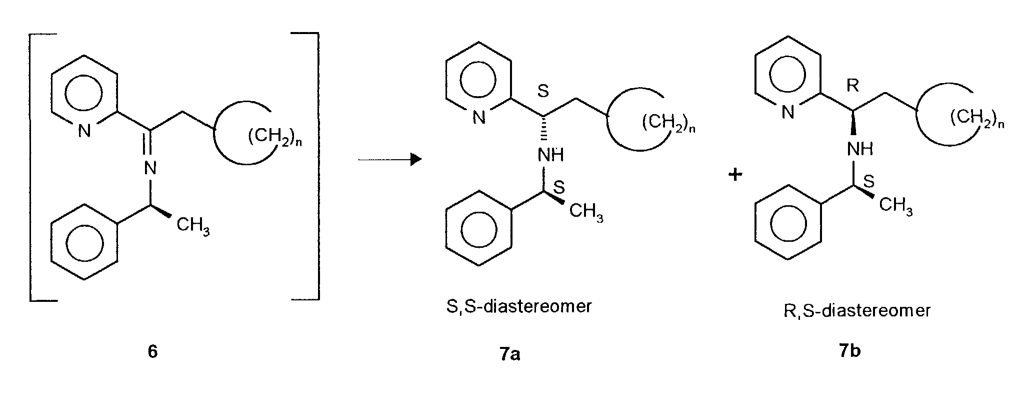
d) das so erhaltene Diastereomerengemisch der Amine mit der allgemeinen Formel 7a und 7b mit Fumar- oder Oxalsäure in einen Niederalkylester einer niederen Carbonsäure bei einer Temperatur von 50°C umsetzt, wobei man die Fumar- oder Oxalsäure vor der Zugabe zu der Diastereomerenmischung in Ethanol löst und so die diastereomeren Amine der allgemeinen Formel 7a und 7b in ihre entsprechenden diastereomeren Fumarate oder Oxalate überführt und das gewünschte Diastereomer anreichert und das entsprechende Diastereomer in Form seines Salzes der allgemeinen Formel 8a oder 8b isoliert; 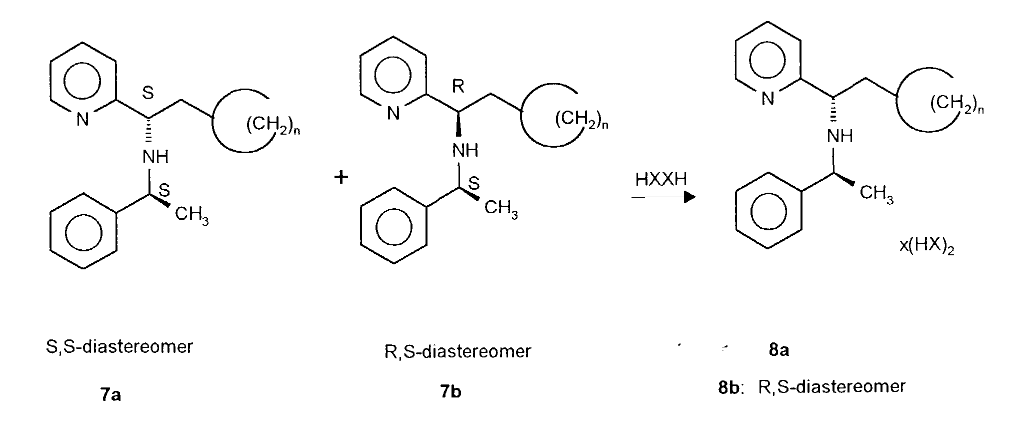
e) das auf diese Art und Weise hergestellte diastereomere Salz der allgemeinen Formel 8a oder 8b in Ethanol löst oder suspendiert und bei einer Temperatur in einem Intervall von 55 bis 60°C löst und in Gegenwart von Palladium auf Kohle (10 % Pd) mit Cyclohexen als Wasserstoffdonor umsetzt und das so debenzylierte Amin der allgemeinen Formel 1a oder 1b mit Ammoniaklösung - bevorzugt mit konzentrierter Ammoniaklösung - aus seinem Salz freisetzt; 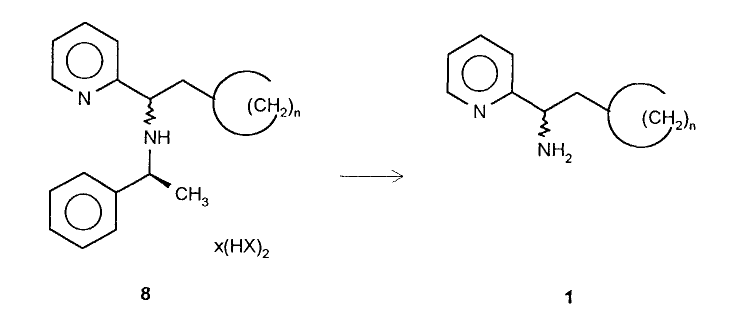
f) das so hergestellte enantiomere Amin der allgemeinen Formel 1a oder 1b gewünschtenfalls mit Oxalsäure in Aceton in das entsprechende Oxalat überführt und isoliert.

4. Verfahren zur Herstellung von optisch reinen Diastereomerensalzen 8a oder 8b eines Pyridylcycloalkylamins der allgemeinen Formel 7a oder 7b 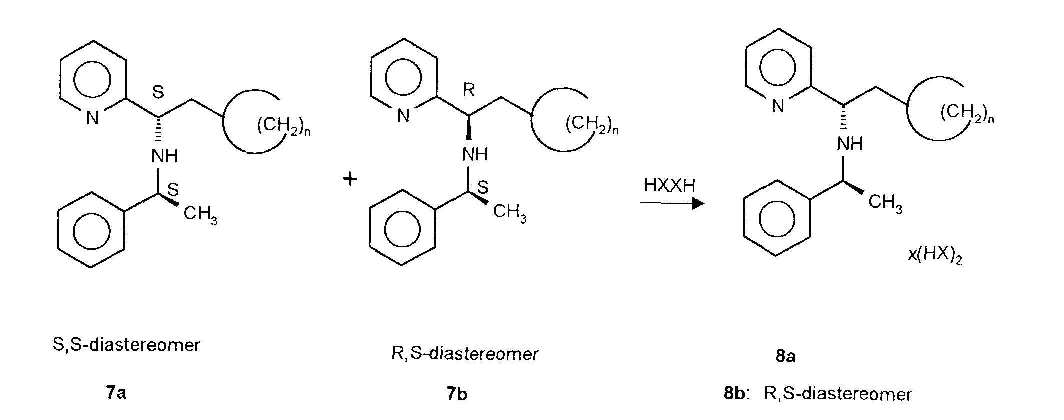 in der n eine ganze Zahl 2, 3, 4, 5, 6 oder 7 bedeutet, dadurch gekennzeichnet, daß man eine Mischung der Diastereoisomoeren 7a und 7b in einem inerten Lösungsmittel mit einer zweibasigen organischen Säure HXXH versetzt und das Salz des einen Diastereomeren von dem Salz des anderen Diastereomeren auf dem Wege der Kristallisation trennt, wobei das auskristallisierende Diastereomerensalz im wesentlich frei von dem löslichlichen Diastereomerensalz ist.

5. Verfahren nach Aspruch 4, dadurch gekennzeichnet, daß als Lösungsmittel ein Niederalkylester (C₁-C₆) einer niederen (C₁-C₆) Carbonsäure eingesetzt wird.

6. Verfahren nach Anspruch 5, dadurch gekennzeichnet, daß als Lösungsmittel Essigsäureethylester eingesetzt wird.

7. Verfahren nach einem der Ansprüche 4 - 6, dadurch gekennzeichnet, daß als Säure Fumar- oder Oxalsäure zur Salzbildung eingesetzt wird.

## Claims

1. Process for preparing enantiomerically pure pyridylcycloalkylethylamines of general formula 1a or 1b 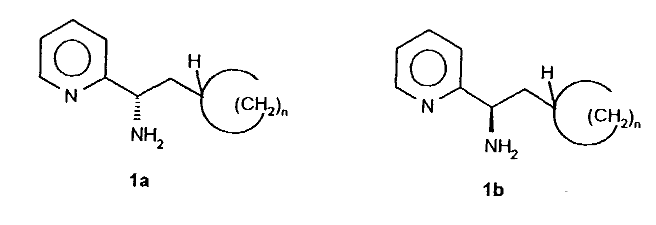 wherein n denotes an integer 2, 3, 4, 5, 6 or 7, characterised in that
a) cyanopyridine (2) is reacted with a halomethylcycloalkane of general formula 3 in the presence of magnesium in an inert reaction medium and after the reaction has ended the reaction mixture is hydrolysed under acidic conditions and the resulting ketone of general formula 4 is isolated; 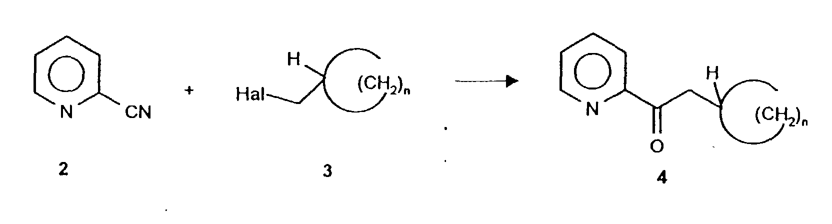
b) the ketone of general formula 4 resulting from the Grignard reaction is reacted with R- or S-phenylethylamine (5) in a dehydration reaction, optionally in the presence of a substance which catalyses dehydration, and the resulting ketimine of general formula 6 is isolated; 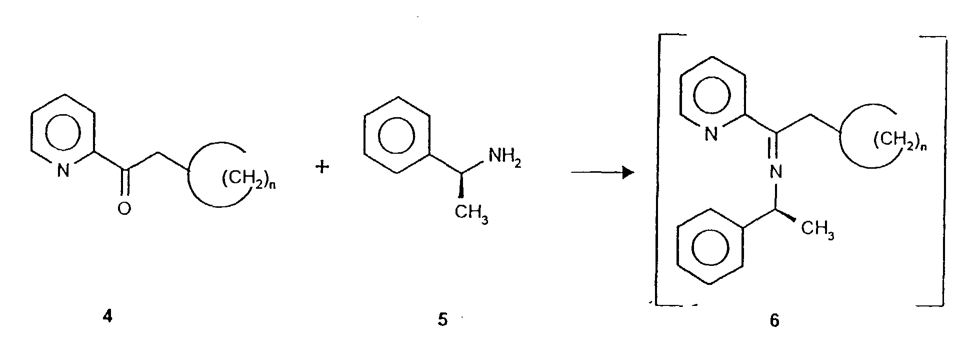
c) the ketimine of general formula 6 resulting from the condensation reaction is reacted with a reducing agent in an inert polar solvent, and after reduction has ended it is adjusted to a pH greater than 8 with an aqueous solution of an alkaline compound and the diastereomers of general formulae 7a and 7b resulting from the reduction are extracted from the reaction mixture using a non-polar solvent and isolated in the form of the mixture of diastereomers; 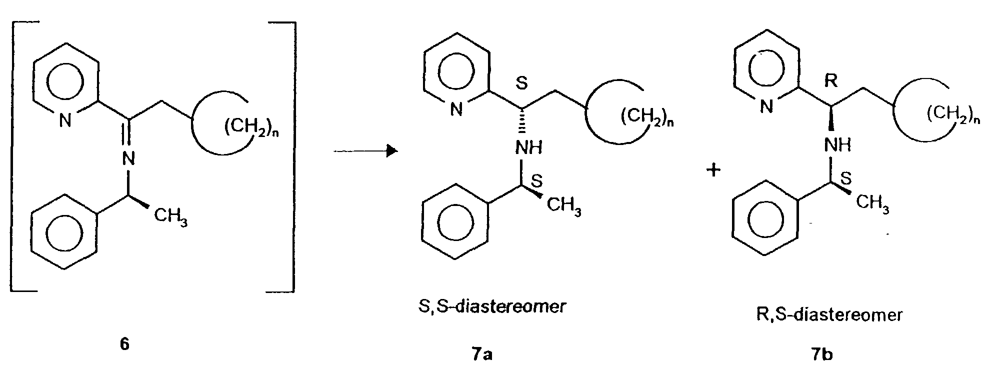
d) the mixture of diastereomers of the amines of general formula 7a and 7b thus obtained is reacted with a suitable acid in an inert solvent, the acid capable of salt formation with the amine in question being dissolved in a lower alcohol (C₁₋₆) before being added to the solution of the mixture of diastereomers, and in this way the amines 7a and 7b are converted into the corresponding diastereomeric salts and thus concentration of the desired diastereomeric salt is obtained and the corresponding diastereomer is isolated in the form of its salt of general formula 8a or 8b; 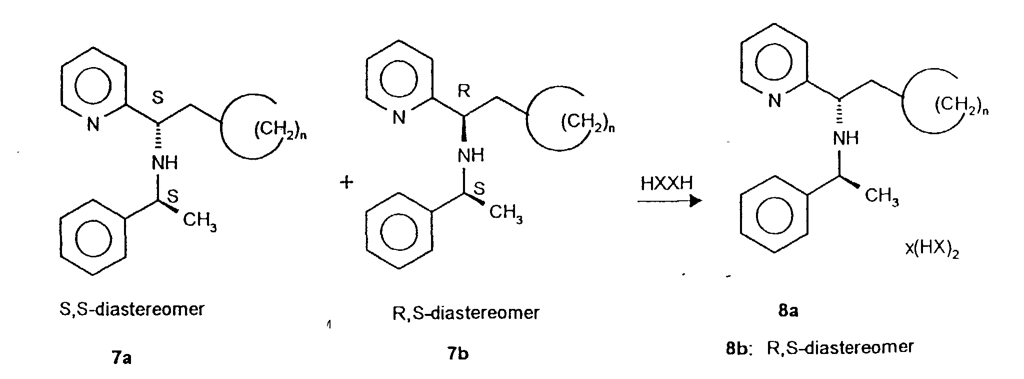
e) the diastereomeric salt 8a or 8b prepared in this way is dissolved or suspended in a polar solvent and reacted at a temperature in the range from 50 to 100°C with a hydrogen donor in the presence of a catalyst capable of catalysing hydrogen transfer and the amine of general formula 1a or 1b debenzylated in this way is released from its salt using a basic compound and isolated; 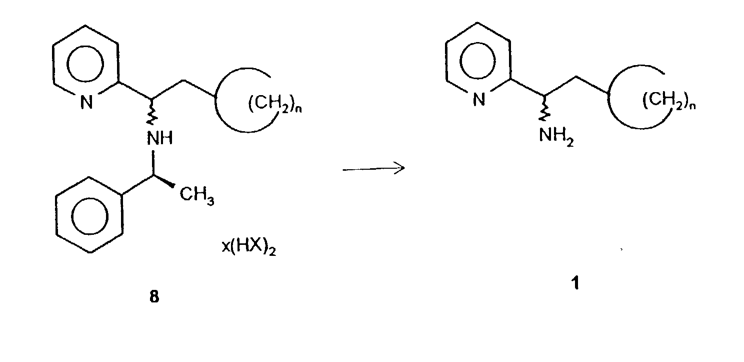
f) the enantiomeric amine of general formula 1a or 1b thus prepared is, if desired, dissolved in a polar solvent and its salt is converted into the corresponding salt using an inorganic or organic acid and isolated.

2. Process according to claim 1, characterised in that
a) cyanopyridine (2) is reacted with a halomethylcycloalkane of general formula 3 in the presence of magnesium in a hydrocarbon or in a dialkylether at a temperature in the range from 40 - 70°C, optionally in the presence of a trialkylhalosilane, and after the reaction has ended the reaction mixture is hydrolysed using a dilute inorganic acid and the resulting ketone of general formula 4 is isolated; 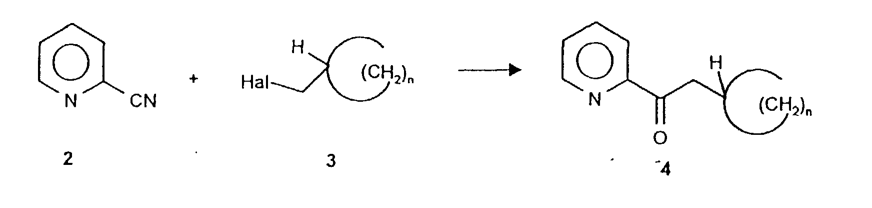
b) the ketone of general formula 4 resulting from the Grignard reaction is reacted with R- or S-phenylethylamine (5) in a dehydration reaction under reflux conditions in a solvent capable of azeotrope formation with water - preferably a haloalkane or an aromatic hydrocarbon - in the presence of a sulphonic acid derivative which catalyses the reaction of condensation or in the presence of silica gel, and the resulting ketimine of general formula 6 is isolated; 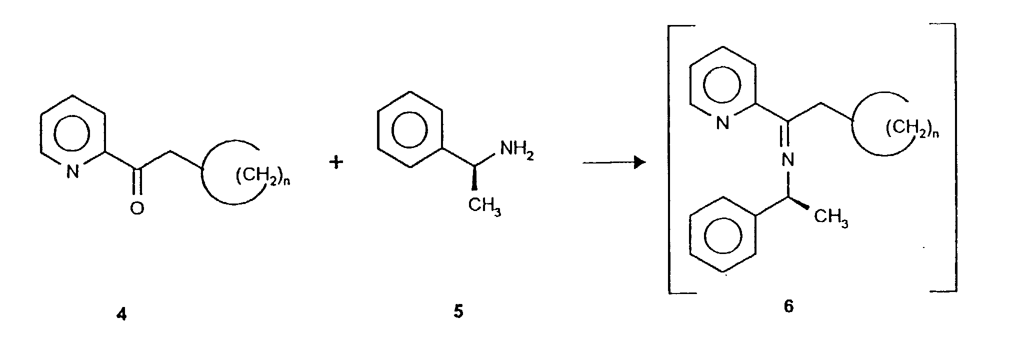
c) the ketimine of general formula 6 resulting from the reaction of condensation is dissolved in an alcohol and reacted with a complex hydride of boron or aluminium at a temperature below 10°C, adjusted to a pH greater than 8 by means of an aqueous solution of an alkali metal hydroxide, and the diastereomers of general formulae 7a and 7b resulting from the reaction of reduction are extracted from the reaction mixture using a dialkylether and isolated in the form of the mixture of diastereomers; 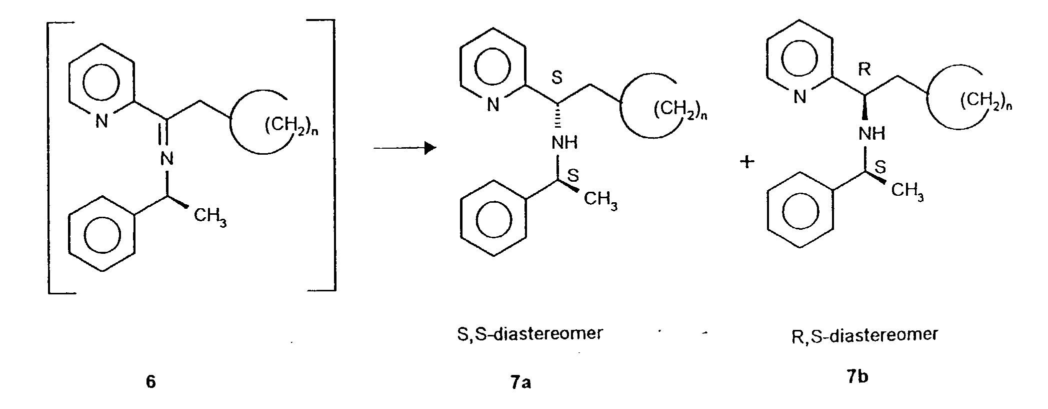
d) the mixture of diastereomers of the amines of general formulae 7a and 7b thus obtained is reacted with an organic acid in a lower alkyl ester of a lower carboxylic acid at a temperature in the range from 40 - 60°C, whilst the acid capable of salt formation with the appropriate amine is dissolved in a lower alcohol (C₁₋₆) before addition to the solution of the mixture of diastereomers, and the diastereomeric amines of general formulae 7a and 7b are converted into the corresponding diastereomeric salts and thus concentration of the desired diastereomeric salt is achieved and the corresponding diastereomer is isolated in the form of its salts of general formula 8a or 8b; 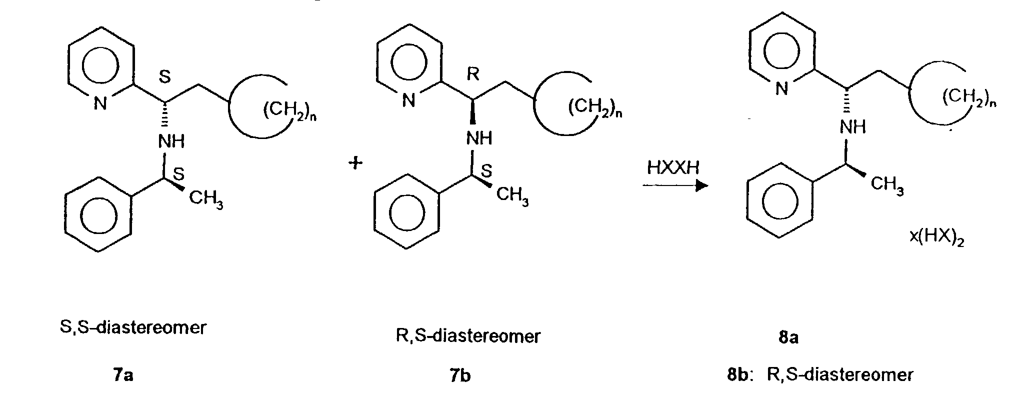
e) the diastereomeric salt of general formula 8a or 8b prepared in this way is dissolved or suspended in a lower alkanol (C₁₋₆) and dissolved at a temperature in the range from 50 to 65°C and reacted with a hydrogen donor in the presence of a platinum-on-charcoal catalyst capable of catalysing hydrogen transfer and the debenzylated amine of general formula 1a or 1b is released from its salt using a nitrogen-containing base, then isolated; 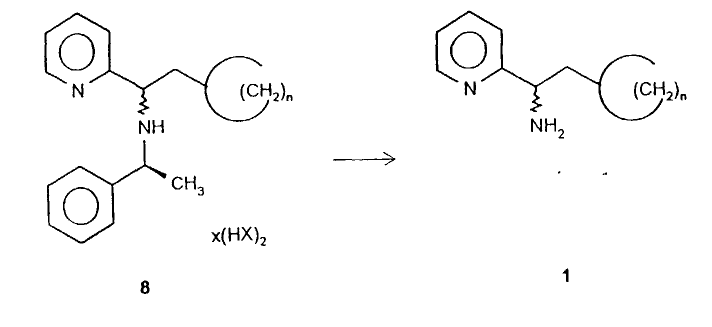
f) the enantiomeric amine of general formula 1a or 1b thus prepared is, if desired, dissolved in a ketone and converted with a carboxylic acid into the corresponding salt, then isolated.

3. Process according to claim 2, characterised in that
a) cyanopyridine (2) is reacted with a bromomethylcycloalkane of general formula 3 in methyl *tert*.butyl ether as the reaction medium at a temperature in the range from 50 to 60°C in the presence of trimethylchlorosilane and after the reaction has ended the reaction mixture is hydrolysed using semi-concentrated hydrochloric acid or sulphuric acid and the resulting ketone of general formula 4 is isolated; 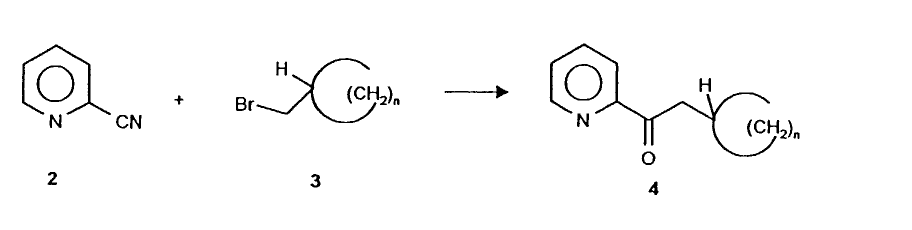
b) the ketone of general formula 4 resulting from the Grignard reaction is dehydrated with R- or S-phenylethylamine (5) in a condensation reaction using azeotropic distillation with toluene and in the presence of silica gel and the ketimine of general formula 6 resulting from this reaction of condensation is isolated; 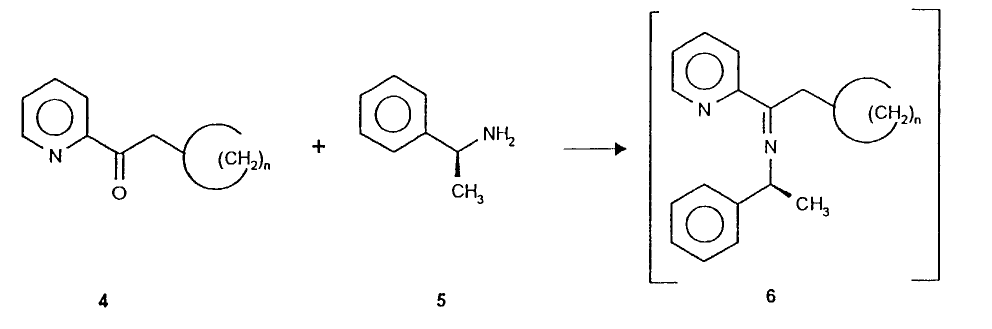
c) the ketimine of general formula 6 resulting from the reaction of condensation is dissolved in ethanol and reacted with sodium tetrahydridoboranate or lithium tetrahydroalanate at a temperature below 10°C, after the reaction has ended the mixture is adjusted to a pH greater than 9 using aqueous sodium hydroxide solution and the diastereomeric amines of general formulae 7a and 7b resulting from the reaction of reduction are extracted with methyl*-tert.*-butylether and isolated in the form of the mixture of diastereomers; 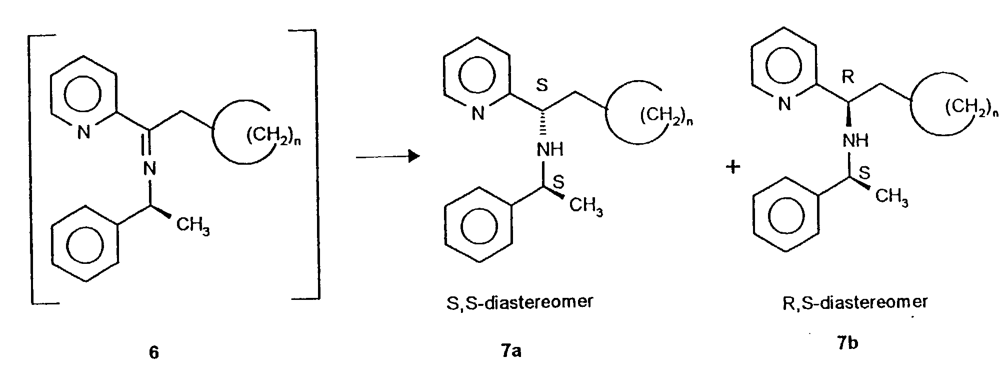
d) the mixture of diastereomers of the amines of general formulae 7a and 7b thus obtained is reacted with fumaric or oxalic acid in a lower alkylester of a lower carboxylic acid at a temperature of 50°C, the fumaric or oxalic acid being dissolved in ethanol before being added to the mixture of diastereomers and in this way the diastereomeric amines of general formula 7a and 7b are converted into their corresponding diastereomeric fumarates or oxalates and the desired diastereomer is concentrated and the corresponding diastereomer is isolated in the form of its salt of general formula 8a or 8b; 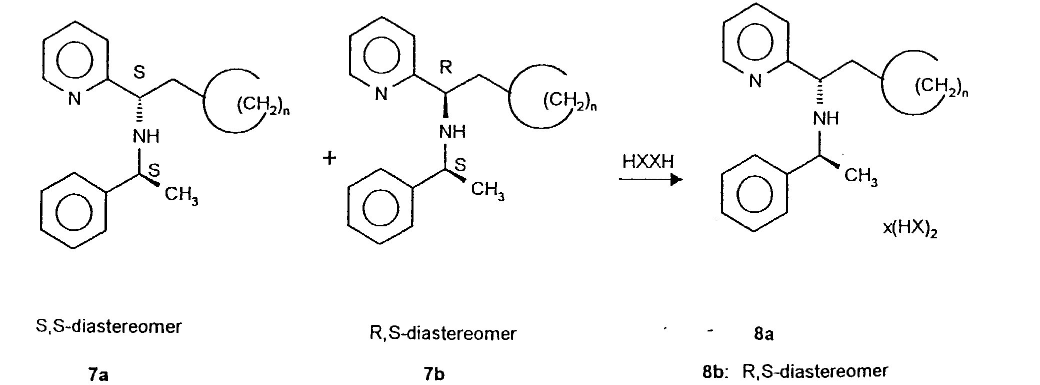
e) the diastereomeric salt of general formula 8a or 8b prepared in this way is dissolved or suspended in ethanol and dissolved at a temperature in the range from 55 to 60°C and reacted with cyclohexene as hydrogen donor in the presence of palladium on charcoal (10% Pd) and the amine of general formula la or 1b debenzylated in this way is released from its salt using ammonia solution, preferably concentrated ammonia solution; 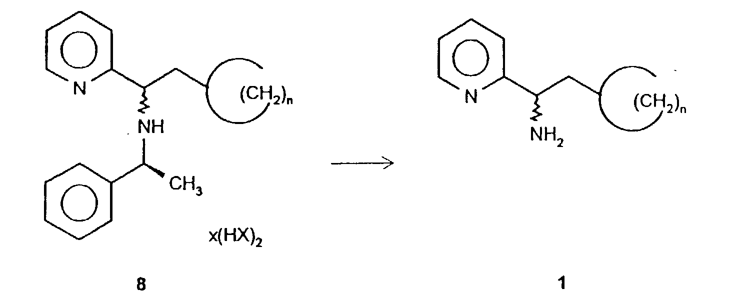
f) the enantiomeric amine of general formula 1a or 1b thus prepared is optionally converted with oxalic acid in acetone into the corresponding oxalate which is then isolated.

4. Process for preparing optically pure diastereomeric salts 8a or 8b of a pyridylcycloalkylamine of general formula 7a or 7b 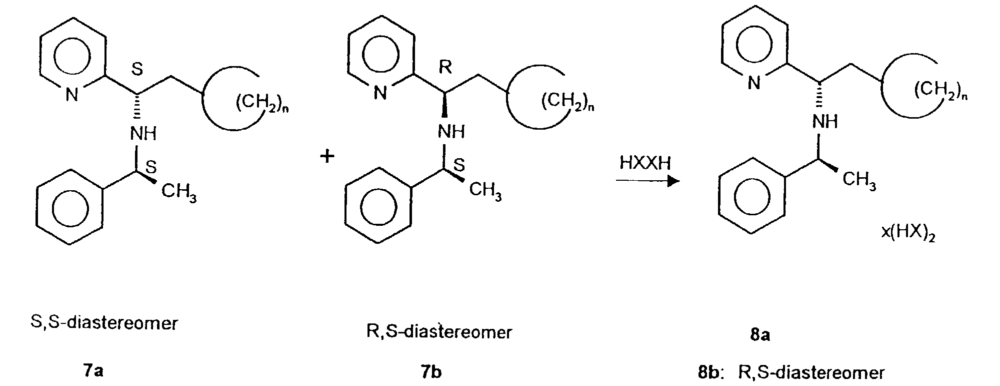 wherein n denotes an integer 2, 3, 4, 5, 6 or 7, characterised in that a mixture of the diastereoisomers 7a and 7b is combined with a dibasic organic salt HXXH in an inert solvent and the salt of one diastereomer is separated from the salt of the other diastereomer by crystallisation, the diastereomeric salt which crystallises out being substantially free from the soluble diastereomeric salt.

5. Process according to claim 4, characterised in that a lower alkyl ester (C₁₋₆) of a lower (C₁₋₆) carboxylic acid is used as the solvent.

6. Process according to claim 5, characterised in that ethyl acetate is used as the solvent.

7. Process according to one of claims 4 to 6, characterised in that fumaric or oxalic acid is used for the salt formation.

## Revendications

1. Procédé de préparation de pyridylcycloalkyléthyl-amines énantiomériquement pures de formule générale 1a ou 1b 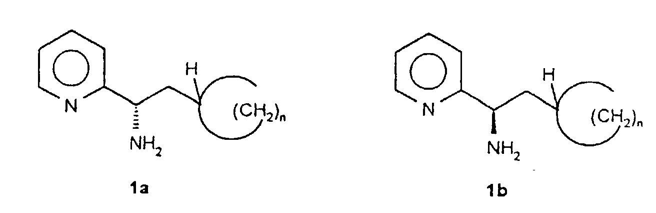 où n représente un nombre entier 2, 3, 4, 5, 6 ou 7, caractérisé en ce que
a) on fait réagir la cyanopyridine (2) avec un halogénométhylcycloalcane de formule générale 3 en présence de magnésium dans un milieu réactionnel inerte et on hydrolyse le mélange réactionnel dans des conditions acides après la fin de la réaction et on isole la cétone de formule générale 4 résultante ; 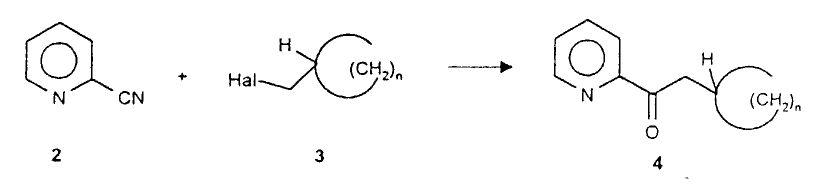
b) on fait réagir la cétone de formule générale 4 résultant de la réaction de Grignard avec la R- ou S-phényléthylamine (5) dans le cadre d'une réaction de déshydratation éventuellement en présence d'une substance catalysant la déshydratation et on isole la cétimine de formule générale 6 résultante ; 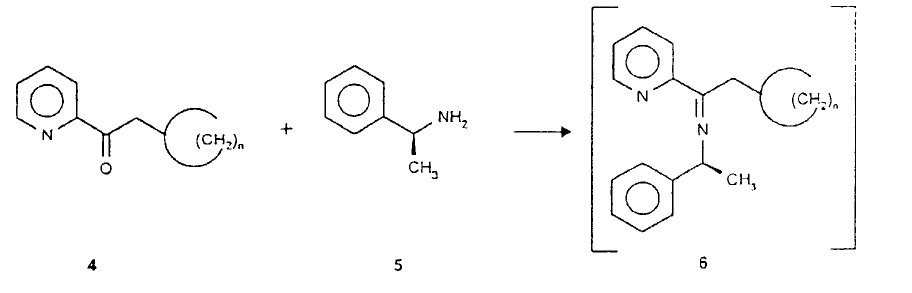
c) on fait réagir avec un réducteur dans un solvant polaire inerte la cétimine de formule générale 6 résultant de la réaction de condensation, après la fin de la réduction on établit un pH supérieur à 8 avec la solution aqueuse d'un composé à réaction alcaline et on extrait du mélange réactionnel avec un solvant non polaire les diastéréoisomères des formules générales 7a et 7b résultant de la réduction et on les isole sous forme de leur mélange de diastéréoisomères ; 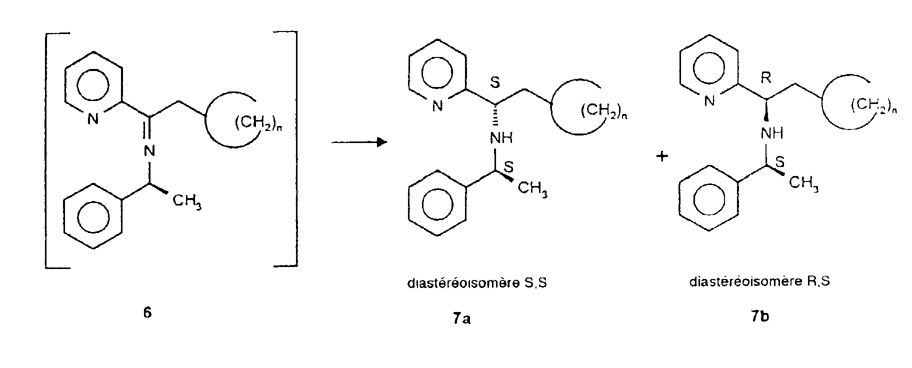
d) on fait réagir avec un acide approprié dans un solvant inerte le mélange de diastéréoisomères des amines des formules générales 7a et 7b ainsi obtenu, en dissolvant dans un alcool inférieur en C₁-C₆ l'acide capable de former un sel avec l'amine respective avant de l'ajouter à la solution du mélange de diastéréoisomères et on convertit ainsi les amines 7a et 7b en les sels diastéréoisomères correspondants et on obtient ainsi un enrichissement du sel diastéréoisomère souhaité respectif et on isole le diastéréoisomère correspondant sous forme de son sel de formule générale 8a ou 8b ; 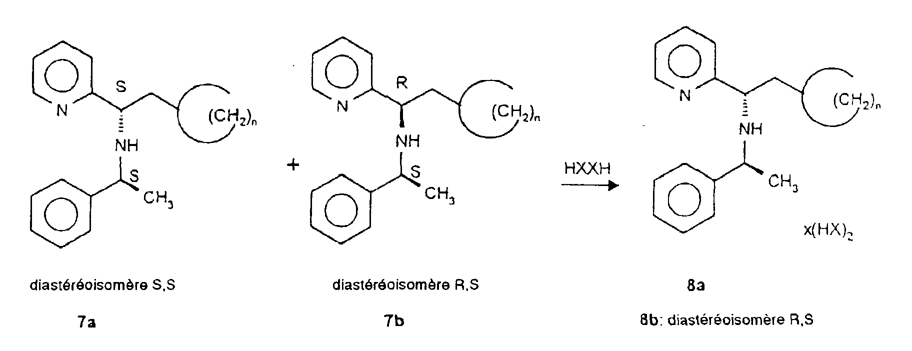
e) on dissout ou met en suspension dans un solvant polaire le sel diastéréoisomère 8a ou 8b préparé de cette manière et on le fait réagir à une température dans le domaine de 50 à 100°C en présence d'un catalyseur capable de catalyse par transfert d'hydrogène avec un donneur d'hydrogène et on libère de son sel avec un composé à réaction basique et isole l'amine ainsi débenzylée de formule générale 1a ou 1b ; 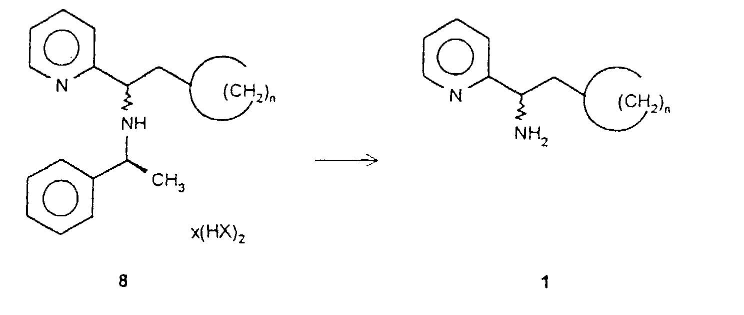
f) si on le souhaite on dissout dans un solvant polaire l'amine énantiomère de formule générale 1a ou 1b ainsi préparée et on convertit son sel avec un acide minéral ou organique en le sel correspondant que l'on isole.

2. Procédé selon la revendication 1 caractérisé en ce que
a) on fait réagir la cyanopyridine (2) avec un halogénométhylcycloalcane de formule générale 3 en présence de magnésium dans un hydrocarbure ou dans un dialkyléther à une température dans un intervalle de 40 - 70°C éventuellement en présence d'un trialkylhalogénosilane et on hydrolyse le mélange réactionnel avec un acide minéral dilué après la fin de la réaction et on isole la cétone de formule générale 4 résultante ; 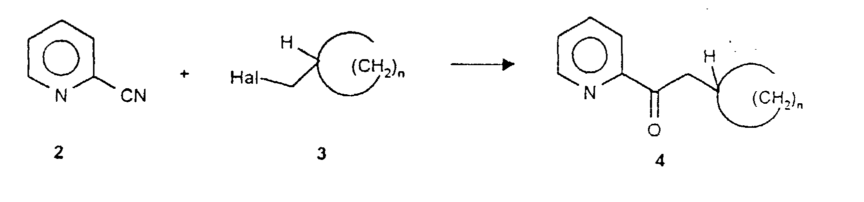
b) on fait réagir la cétone de formule générale 4 résultant de la réaction de Grignard avec la R- ou S-phényléthylamine (5) dans le cadre d'une réaction de déshydratation dans des conditions de reflux dans un solvant capable de former un azéotrope avec l'eau, de préférence un halogénoalcane ou un hydrocarbure aromatique, en présence d'un dérivé d'acide sulfonique catalysant la réaction de condensation ou en présence de gel de silice et on isole la cétimine de formule générale 6 qui en résulte; 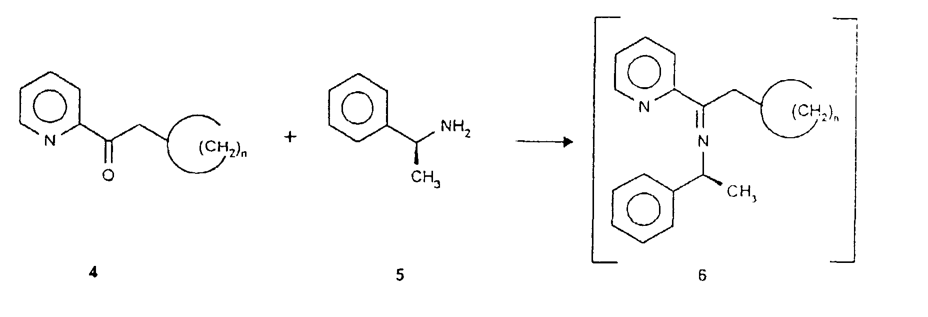
c) on dissout dans un alcool la cétimine de formule générale 6 résultant de la réaction de condensation et on la fait réagir avec un hydrure complexe du bore ou de l'aluminium à une température inférieure à 10°C, on établit un pH supérieur à 8 avec la solution aqueuse d'un hydroxyde alcalin et on extrait du mélange réactionnel avec un dialkyléther les diastéréoisomères des formules générales 7a et 7b résultant de la réaction de réduction et on les isole sous forme de leur mélange de diastéréoisomères ; 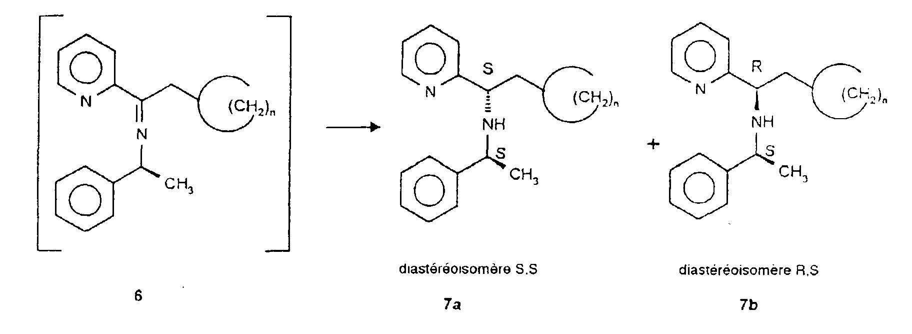
d) on fait réagir avec un acide organique dans un alkylester inférieur d'un acide carboxylique inférieur à une température dans un intervalle de 40 - 60°C le mélange de diastéréoisomères des amines des formules générales 7a et 7b ainsi obtenu, en dissolvant dans un alcool inférieur en C₁-C₆ l'acide capable de former un sel avec l'amine respective avant de l'ajouter à la solution du mélange de diastéréoisomères et on convertit les amines diastéréoisomères des formules générales 7a et 7b en les sels diastéréoisomères correspondants et on obtient ainsi un enrichissement du sel diastéréoisomère souhaité et on isole le diastéréoisomère correspondant sous forme de ses sels de formule générale 8a ou 8b ; 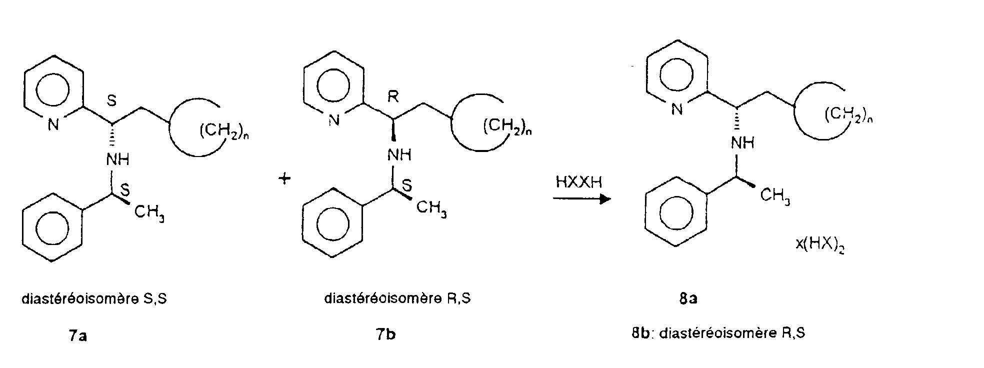
e) on dissout ou met en suspension dans un alcool inférieur (alcool en C₁-C₆) le sel diastéréoisomère de formule générale 8a ou 8b préparé de cette manière et on le fait réagir à une température dans le domaine de 50 à 65°C et en présence d'un catalyseur de platine sur charbon capable de catalyse par transfert d'hydrogène avec un donneur d'hydrogène et on libère de son sel avec une base azotée et isole l'amine débenzylée de formule générale 1a ou 1b ; 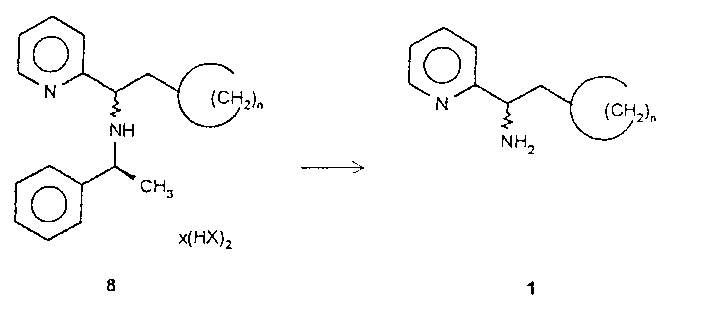
f) si on le souhaite on dissout dans une cétone l'amine énantiomère de formule générale 1a ou 1b ainsi préparée et on la convertit avec un acide carboxylique en le sel correspondant que l'on isole.

3. Procédé selon la revendication 2 caractérisé en ce que
a) on fait réagir la cyanopyridine (2) avec un bromométhylcycloalcane de formule générale 3 dans le méthyl-*tert.*-butyléther comme milieu réactionnel à une température dans un intervalle de 50 à 60°C en présence de triméthylchlorosilane et on hydrolyse le mélange réactionnel avec l'acide chlorhydrique ou sulfurique semiconcentré après la fin de la réaction et on isole la cétone de formule générale 4 résultante ; 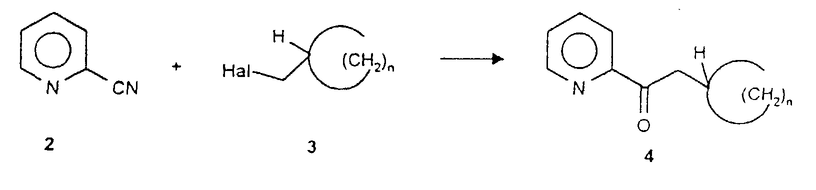
b) on déshydrate la cétone de formule générale 4 résultant de la réaction de Grignard avec la R- ou S-phényléthylamine (5) dans le cadre d'une réaction de condensation par distillation azéotropique avec le toluène et en présence de gel de silice et on isole la cétimine de formule générale 6 résultant de cette réaction de condensation ; 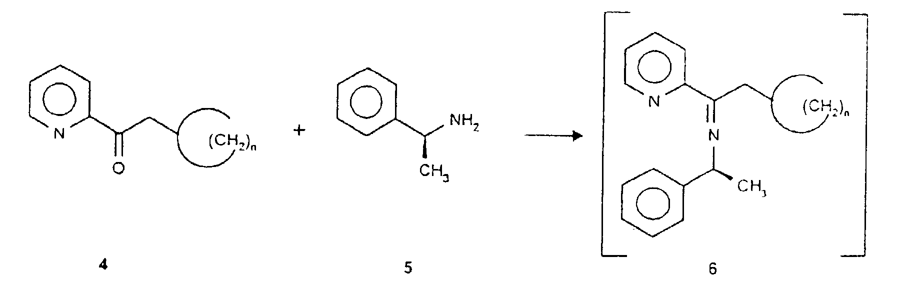
c) on dissout dans l'éthanol la cétimine de formule générale 6 résultant de la réaction de condensation et on la fait réagir avec le tétrahydruroboranate de sodium ou le tétrahydroalanate de lithium à une température inférieure à 10°C, après la fin de la réaction on établit un pH supérieur à 9 avec de la lessive de soude aqueuse et on extrait avec le méthyl*-tert.*-butyléther les amines diastéréoisomères des formules générales 7a et 7b résultant de la réaction de réduction et on les isole sous forme de leur mélange de diastéréoisomères ; 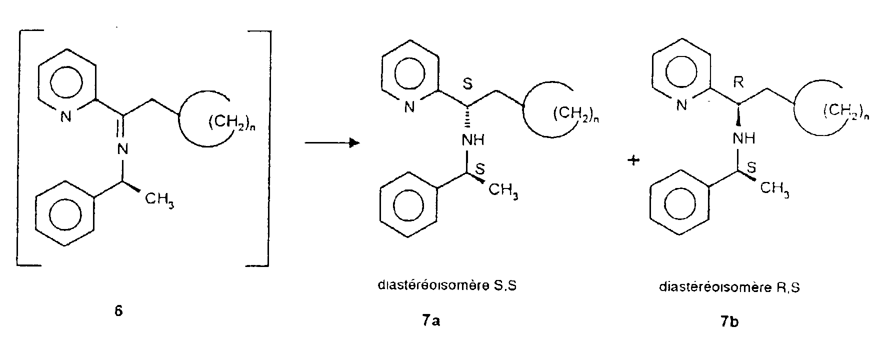
d) on fait réagir avec l'acide fumarique ou oxalique dans un alkylester inférieur d'un acide carboxylique inférieur à une température de 50°C le mélange de diastéréoisomères des amines des formules générales 7a et 7b ainsi obtenu, en dissolvant dans l'éthanol l'acide fumarique ou oxalique avant de l'ajouter au mélange de diastéréoisomères et on convertit les amines diastéréoisomères des formules générales 7a et 7b en leurs fumarates ou oxalates diastéréoisomères correspondants et on enrichit le diastéréoisomère souhaité et on isole le diastéréoisomère correspondant sous forme de son sel de formule générale 8a ou 8b ; 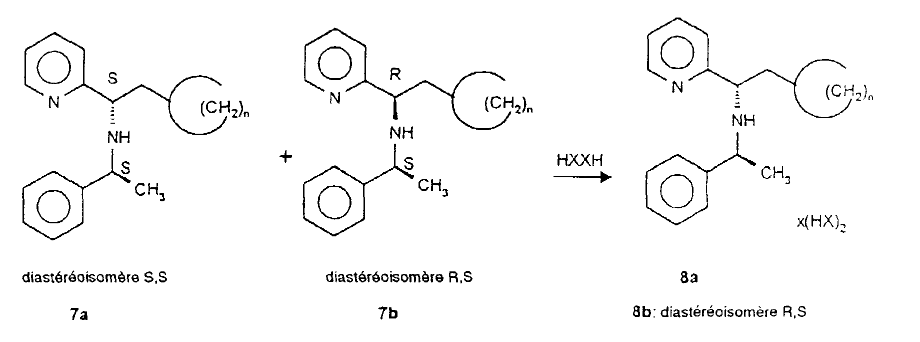
e) on dissout ou met en suspension dans l'éthanol le sel diastéréoisomère de formule générale 8a ou 8b préparé de cette manière et on le dissout à une température dans un intervalle de 55 à 60°C et on le fait réagir en présence de palladium sur charbon (10 % de Pd) avec le cyclohexène comme donneur d'hydrogène et on libère de son sel avec une solution d'ammoniac, de préférence avec une solution d'ammoniac concentrée, l'amine ainsi débenzylée de formule générale 1a ou 1b ; 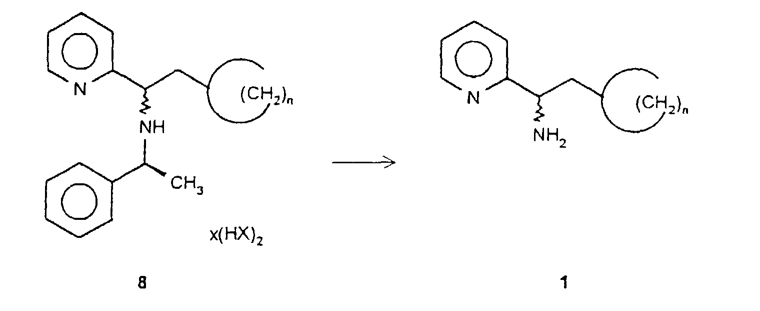
f) si on le souhaite on convertit l'amine énantiomère de formule générale 1a ou 1b ainsi préparée avec l'acide oxalique dans l'acétone en l'oxalate correspondant que l'on isole.

4. Procédé de préparation de sels diastéréoisomères 8a ou 8b optiquement purs d'une pyridylcycloalkylamine de formule générale 7a ou 7b 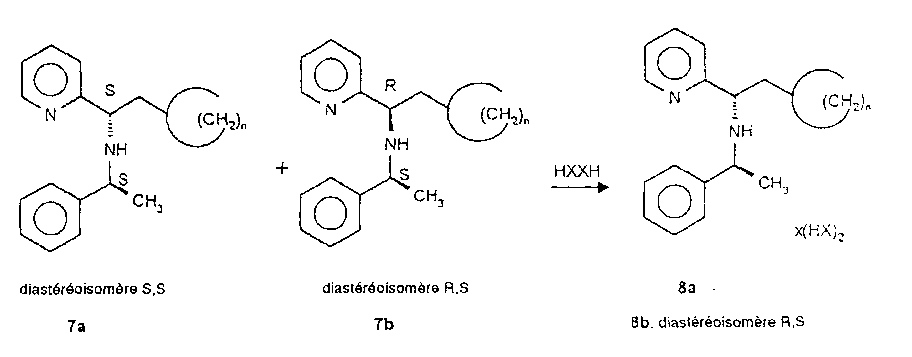 où n représente un nombre entier 2, 3, 4, 5, 6 ou 7, caractérisé en ce que l'on ajoute un acide organique difonctionnel HXXH à un mélange des diastéréoisomères 7a et 7b dans un solvant inerte et on sépare le sel d'un diastéréoisomère du sel de l'autre diastéréoisomère par cristallisation, le sel diastéréoisomère qui cristallise étant sensiblement exempt du sel diastéréoisomère soluble.

5. Procédé selon la revendication 4 caractérisé en ce que l'on utilise comme solvant un alkylester inférieur en C₁-C₆ d'un acide carboxylique inférieur en C₁-C₆.

6. Procédé selon la revendication 5 caractérisé en ce que l'on utilise l'acétate d'éthyle comme solvant.

7. Procédé selon l'une des revendications 4 - 6 caractérisé en ce que l'on utilise l'acide fumarique ou l'acide oxalique comme acide pour la salification.
